# EUROPEAN PATENT SPECIFICATION

(11) **EP 2 858 549 B1**
(45) Date of publication and mention of the grant of the patent: **26.06.2019**
(21) Application number: 13801154.9
(22) Date of filing: 23.05.2013
(51) Int. Cl.: A61B 1/04

(54) **CAPSULE ENDOSCOPIC DOCKING SYSTEM**
ANDOCKSYSTEM FÜR KAPSELENDOSKOPIE
SYSTÈME D'ANCRAGE ENDOSCOPIQUE À CAPSULE

(30) Priority: 09.06.2012 US 201261657747 P
(43) Date of publication of application: 15.04.2015
(73) Proprietor: Wilson, Gordon, San Francisco, California 94114 (US); Wang, Kang-Huai, Saratoga, California 95070 (US); Capso Vision, Inc., Saratoga, CA 95070 (US)
(72) Inventor: WILSON, Gordon, San Francisco, California 94114 (US); WANG, Kang-Huai, Saratoga, California 95070 (US)
(74) Representative: Papula Oy
(86) International application number: PCT/US2013/042490
(87) International publication number: WO 2013/184386

(56) References cited:
- WO-A1-2007/082348
- WO-A1-2011/111433
- CN-U- 201 641 948
- CN-U- 201 668 367
- JP-A- 2007 185 521
- JP-A- 2008 173 322
- JP-A- 2011 139 796
- US-A1- 2003 023 150
- US-A1- 2007 025 809
- US-A1- 2008 311 795
- US-A1- 2009 299 140
- US-A1- 2010 062 615
- US-A1- 2011 239 714
- US-A1- 2011 249 105
- US-A1- 2011 279 081
- US-B1- 6 623 500

## Description

### TECHNICAL FIELD

An embodiment relates to diagnostic imaging inside the human body. In particular, the embodiment relates to a docking station for supplying power to and retrieving data from a capsule camera.

### BACKGROUND AND RELATED ART

Devices for imaging body cavities or passages in vivo are known in the art and include endoscopes and autonomous encapsulated cameras. Endoscopes are flexible or rigid tubes that pass into the body through an orifice or surgical opening, typically into the esophagus via the mouth or into the colon via the rectum. An image is formed at the distal end using a lens and transmitted to the proximal end, outside the body, either by a lens-relay system or by a coherent fiber-optic bundle. A conceptually similar instrument might record an image electronically at the distal end, for example using a CCD or CMOS array, and transfer the image data as an electrical signal to the proximal end through a cable. Because of the difficulty traversing a convoluted passage, endoscopes cannot reach the majority of the small intestine and special techniques and precautions, that add cost, are required to reach the entirety of the colon. An alternative in vivo image sensor that addresses many of these problems is capsule endoscope. A camera is housed in a swallowable capsule, along with a radio transmitter for transmitting data, primarily comprising images recorded by the digital camera, to a base-station receiver or transceiver and data recorder outside the body. Another autonomous capsule camera system with on-board data storage was disclosed in the US Patent US2007098379 A1.

For the above in vivo devices, a large amount of image data is collected as it passes through a lumen in human body such as the gastrointestinal (GI) tract. The images captured, along with other information, are stored in the on-board archival memory inside the capsule camera. The archival memory may come in various forms of non- volatile memories. After the capsule camera is excreted or otherwise removed from the body, it is retrieved to recover the data stored on-board. In a conventional approach, it would require a fairly expensive data access system that includes opening the capsule and docking to it. Because of the requirement to open the capsule and align the contact pins to pads in the capsule, some degree of mechanical complexity is inevitable. Therefore, such tasks usually are performed by specially trained persons. It is desirable to develop a new system that allows data retrieval from the capsule camera without opening the sealed enclosure and with simplified operation for clinicians accessing the capsule data.

A publication JP 2008 173322 A (hoya crop 31 July 2008 (2008-07-31) discloses background art which may be useful for understanding the background.

### BRIEF SUMMARY

A capsule endoscopic system is disclosed, wherein the system comprises a capsule endoscope, a docking station, second circuits and contact means to cause the second circuits connected to first circuits inside the capsule endoscope when the capsule endoscope is docked in the docking station in a first rotational orientation and in a second rotational orientation. The capsule endoscope comprises a capsule housing, the first circuits, and one or more first contacts disposed fixedly on or through the capsule housing, wherein the first contacts are connected to the first circuits. The docking station comprises one or more second contacts. The docking station also includes a docking bay to receive the capsule endoscope. The second contacts are configured to contact the first contacts and to cause the first circuits to connect to the second circuits when the capsule endoscope is docked in the first rotational orientation and the second rotational orientation.

The first contacts may comprise one or more feedthrough connectors located at one end of the capsule endoscope. The feedthrough connectors are substantially flush with exterior surface of the capsule housing. The feedthrough connectors or the second contacts are configured to be circular symmetric around a longitudinal axis through a center of the capsule endoscope. In one embodiment, the feedthrough connectors comprise studs and the second contacts comprise one or more annular connectors. Furthermore, the annular connectors may comprise a series of spokes extended radially inward from an annular outer ring. In another embodiment not part of the present invention, the feedthrough connectors comprise one or more concentric ring connectors and the second contacts comprise one or more plunger contacts. Furthermore, the feedthrough connectors comprise a center connector located at a center of said one or more concentric ring connectors.

In other embodiments, the first connectors are located at a wall section of the capsule endoscope. The feedthrough connectors or the second contacts are configured to be circular symmetric around a longitudinal axis through a center of the capsule endoscope. In one embodiment not part of the present invention, the feedthrough connectors comprise studs and the second contacts comprise multiple plunger contacts arrayed in a circle around the capsule endoscope. In another embodiment not part of the present invention, the feedthrough connectors comprise one or more concentric ring connectors and the second contacts comprise one or more plunger contacts.

One aspect of the embodiment addresses power control for the capsule endoscope. The capsule endoscope further comprises a battery and a diode inside the capsule housing, wherein power is provided from or through the docking station to the capsule endoscope through a first connector and a second connector, and the diode is connected to the first connector so as to block current from flowing from the battery to the first connector while allowing current to flow from the first connector to the first circuits. In another arrangement, a switch is used instead, wherein the switch is connected to the first connector so as to block current from flowing from the battery to the first connector while the capsule endoscope is not docked in the docking station.

In another embodiment, the contact means comprises one or more first contacts disposed fixedly on or through the capsule housing, one or more second contacts in the docking stations and wherein the second contacts are configured to contact the first contacts and to cause the first circuits to connect to the second circuits when the capsule endoscope is docked in or rotated to an aligned rotational orientation. Two detection contacts are located in the docking station, wherein said two detection contacts are configured to cause connection with a location contact disposed on exterior surface of the capsule housing when the capsule endoscope is at the aligned rotational orientation in the docking bay. The first contacts are connected to the first circuits and the second contacts are coupled to the second circuits.

### BRIEF DESCRIPTION OF THE DRAWINGS

Figs. 1A-1D illustrate an example of capsule camera with feedthrough interconnect with a docking station according to an embodiment.
Figs. 2A-2B illustrate a variation of capsule camera with feedthrough interconnect.
Figs. 3 illustrates an example of capsule camera with feedthrough interconnect with a docking station, where the system uses an alternative arrangement of the feedthrough interconnect.
Figs. 4A-4B illustrate illustrates an example of feedthrough interconnect arrangement having annular contacts around the middle of the capsule and mating ring contacts in the docking station.
Figs. 5A-5B illustrate an alternative arrangement of feedthrough interconnect, where the docking station has multiple plunger contacts arrayed in a circle about the capsule at angular intervals Θ and the capsule has a feedthrough contact.
Fig. 6A illustrates an exemplary schematic of the capsule, where a diode is used to protect from current flow through the bodily tissues or through liquids in the GI tract.
Fig. 6B illustrates an exemplary schematic of the capsule, where a switch activated by externally applied voltage is use to protect from current flow through the bodily tissues or through liquids in the GI tract.
Fig. 6C illustrates an exemplary schematic of the capsule, where a transistor is use to protect from current flow through the bodily tissues or through liquids in the GI tract.
Fig. 7A-7B illustrates embodiment, where the capsule and the docking station contacts both lack circular symmetry.
Fig. 8A-7B illustrates embodiment, where the feedthrough interconnect is slightly different from the system in Figs. 7A-7B.

### DETAILED DESCRIPTION

It will be readily understood that the components of the present invention, as generally described and illustrated in the figures herein, may be arranged and designed in a wide variety of different configurations. Thus, the following more detailed description of the embodiments of the systems and methods of the present invention, as represented in the figures, is not intended to limit the scope of the invention, as claimed, but is merely representative of selected embodiments of the invention. Reference throughout this specification to "one embodiment," "an embodiment," or similar language means that a particular feature, structure, or characteristic described in connection with the embodiment may be included in at least one embodiment of the present invention. Thus, appearances of the phrases "in one embodiment" or "in an embodiment" in various places throughout this specification are not necessarily all referring to the same embodiment.

Furthermore, the described features, structures, or characteristics may be combined in any suitable manner in one or more embodiments. One skilled in the relevant art will recognize, however, that the invention can be practiced without one or more of the specific details, or with other methods, components, etc. In other instances, well-known structures, or operations are not shown or described in detail to avoid obscuring aspects of the invention. The illustrated embodiments of the invention will be best understood by reference to the drawings, wherein like parts are designated by like numerals throughout. The following description is intended only by way of example, and simply illustrates certain selected embodiments of apparatus and methods that are consistent with the invention as claimed herein.

Systems for receiving data from a capsule endoscope or transmitting data to a capsule endoscope have been disclosed. US 2003/0023150 describes a capsule that may be opened after excretion exposing electrical contacts. A "rewriting unit" or docking station has corresponding contacts that mate with the capsule contacts for data transfer via electrical signals. US 7,983,458, assigned to Capso Vision, discloses "a connector at the output port" which provides "a hermetic or near-hermetic bulkhead feedthrough imbedded in the housing wall, such than an electrical connection can be made between the connector at the output port of the capsule and its mate at the upload device, without breaking the seal." The connection made can provide power to the capsule as well as transmitting data from and to the capsule.

In order to supply power to the capsule by electrical conduction, a minimum of two contacts is required. Using certain modulation formats, data can be transferred serially via these contacts as well. More commonly, at least one additional contact is utilized for data transfer. The data clock may be recovered in the docking station receiver. Alternatively, a separate contact may be used to transmit the clock signal or data may be transferred asynchronously, for example to/from a universal asynchronous receiver/transmitter (UART). If the capsule has feedthrough contacts that terminate on the capsule's surface, then the capsule does not need to be cut open in order to make electrical contact to the capsule electronics and transfer data from or to the capsule.

The capsule housing should have a smooth surface so that it is easy to swallow and passes easily and safely through the gastrointestinal (GI) system with minimal drag and without snagging on or puncturing bodily tissues in the GI tract. A smooth surface also limits fouling of the capsule by debris in the GI tract, which could impair imaging of the mucosal surfaces by one or more cameras in the capsule. Moreover, capsules typically have circular symmetry to simplify their manufacture and in order to maximize the interior volume, with a limit on the largest lateral dimension imposed by the need to easily swallow the capsule.

If the electrical contact points on the capsule and in the docking station lack approximate circular symmetry about the capsule's longitudinal axis, then the capsule must be aligned rotationally in order to make the correct connection to the docking station. If the capsule housing's morphology has circular symmetry, then no key can be provided on the capsule to mechanically set the rotational alignment of the capsule in the docking station. The user may make a visual alignment to a mark on the capsule, but this may be difficult to do with sufficient accuracy given the small size of the capsule (typically <12mm in diameter).

Alternatively, a sensor (e.g. a vision system or inductive or optical proximity sensor) in the docking station may be used to automatically sense the capsule rotation. The capsule may be rotated manually or by an electromechanical mechanism until the sensor determines that the correct alignment is achieved. Including such a sensor increases the cost of the system, however.

Therefore it is desirable to make either the contacts on the capsule surface and/or those in the docking station with approximate circular symmetry about the longitudinal axis of the capsule. The contacts do not need to have perfect circular symmetry, but the connection between the contacts must be maintained regardless of the relative rotational angle between the capsule and the docking station. For example, a contact with approximate circular symmetry may have an annular shape and the annulus may have small gaps, regularly or irregularly spaced, as long as the gaps are smaller than the width of the mating contact (e.g. a pin) in the tangential direction. Moreover, since one contact in a mating pair is usually compliant, the point of contact need not remain in a plane as the capsule is rotated. As the capsule is rotated, the contact point between two mating contacts should follow a path, whose projection onto a plane normal to the compliant contact's direction of motion does not deviate from a circle more than does either contact. The path may have gaps in it, but these should be smaller than the width, tangent to the capsule rotation, of that contact which lacks approximate circular symmetry. A contact satisfying these conditions has "approximate circular symmetry". If an interconnection is made between contacts on the capsule and those in the docking station for any rotational orientation of the capsule, then one or both sets of contacts has "approximate circular symmetry".

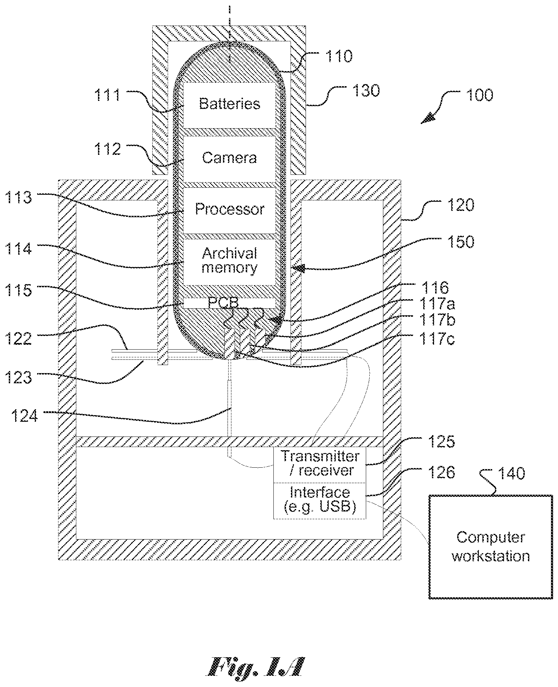

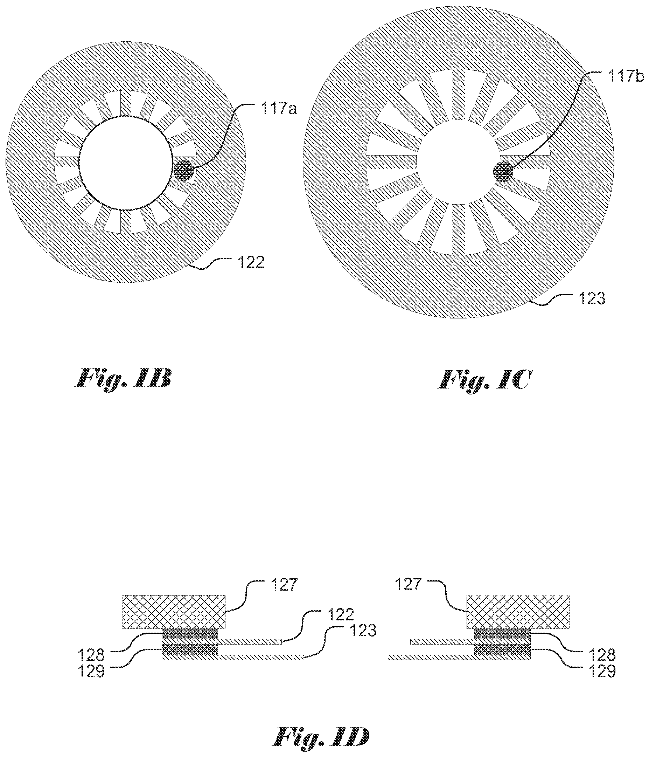

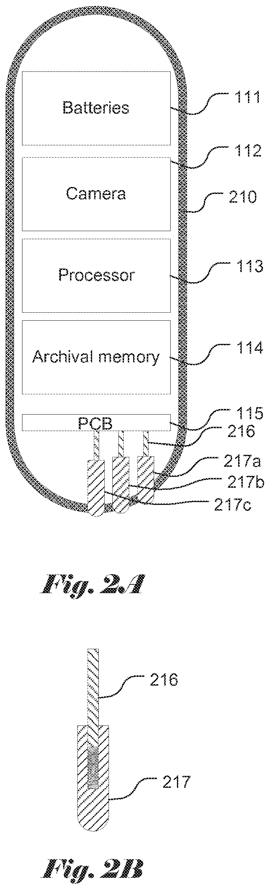

One capsule endoscope and docking station configuration embodying the feedthrough interconnect according to the embodiment is shown in Fig. 1A, where the capsule camera 110 is anchored in the docking station base 120 with a cover 130 closed. The capsule camera 110 may comprise batteries 111, camera module 112, processor 113, archival memory 114, and printed circuit board (PCB) 115. On the other hand, the docking station 100 comprises a base enclosure 120 and a cover 130. The docking station may be connected to a computer workstation 140 for storage and presentation of data. The base enclosure hosts annular connectors 122 and 123, spring-loaded (pogo) pin 124, transmitter/receiver 125, and interface 126. While transmitter/receiver 125 is shown in Fig. 1A as part of the docking station, transmitter/receiver 125 may also be external to the docking station. In this case, the interface (126) may become connection terminals or connectors on the docking station to provide contact to the capsule endoscope. As mentioned before, the docking station may need to supply power to the capsule endoscope. Therefore, the docking station may include a power source or provide connectivity between an external power source and the capsule endoscope. The capsule endoscope has two ends along the longitudinal direction. Contacts may be disposed on one end of the capsule endoscope. The interconnect system is shown with three contacts 117a-c, although the number could be any number greater than one. Three studs pass through the capsule housing to form feedthrough contacts (electrodes). The studs include an electrically conductive material such as a metal, semiconductor, carbon, or conductive metal oxide. In a preferred embodiment, the studs are coated with a noble metal such as gold or platinum, at least on the contact surfaces. These materials are resistant to corrosion and are biocompatible. One end of each stud has a surface that is flush to the capsule, in the example of Fig. 1A. However, the studs need not be exactly flush to the housing outer surface as long as their recess or protrusion is small and the outer surface of the capsule, including the studs, is smooth. The recessed area in the docking station forms a docking bay (150) to receive the capsule endoscope. Fig. IB shows a bottom view of the annular connector 122 alone with a possible stud 117a contact position indicated.. Fig. 1C shows a bottom view of the annular connector 123 alone with a possible stud 117b contact position indicated. Fig. ID illustrates an example of supporting structure for the annular connectors 122 and 123, where block 127 corresponds to a part of the enclosure structure and elements 128 and 129 are compliant spacers. The contact means as shown in Figs. 1A-1D will cause desired electrical connection between the circuits in the capsule endoscope and the transmitter/receiver. The studs might have spherical tips, for example and may also slightly extend beyond the housing surface of the capsule camera 210 as shown in Fig. 2A.

The capsule may be made water tight by creating a seal around each stud. The housing may consist of two or three sections that are pushed together and sealed by an adhesive or welding process during the capsule manufacturing. For example, the housing may comprise a cylindrical center section with two end caps. One housing section may be fabricated with a hole for each stud. The studs may be inserted and an adhesive may be applied to affix the studs and form a seal. Heat may be used to soften or locally melt the housing in order to form a seal around the studs. Alternately, a gasket may be provided around each stud. The capsule housing section may be formed with the studs pre-positioned using mold-in place technology to mold a housing material such as a thermoplastic around the studs.

Each stud (117a-c) must electrically connect to the capsule endoscope internal electronic system so that power can be supplied to the endoscope and data may be transferred from or to memory in the endoscope. Such memory could include an archival non-volatile memory 114 (e.g. flash memory) and/or a volatile memory contained in the processor (e.g. RAM). In Fig. 1A, a flexible interconnect 116 is shown connecting the studs to a printed circuit board (PCB) 115. The interconnect could comprise spring-loaded pins; flexible metal tabs; thin wires in an elastomer matrix; an elastomer coated with or filled with traces (insulated conduction paths) made of carbon, silver, or gold; or any other compliant conductive interconnect system. Connections could also be made by soldering wires from the studs 117a-c to the PCB 115. In order to simplify assembly, the interconnect in the capsule may be configured with a sufficient degree of circular symmetry such that the rotational orientation of one capsule section to another is not critical.

Alternatively, the studs 217a-c themselves could have compliant members, the tips of which contact either contact pads on the PCB directly or contact electrodes connected to the PCB as shown in Figs. 2A-2B. The studs might be spring loaded pins 216 with a plunger in a cylinder like a pogo pin. They may instead have flexible extensions or tabs that contact the PCB and deflect upon doing so.

In Fig. 1A, the connectors in the docking station form nearly complete rings contacting the surface of the capsule. The center connector may be a compliant pin, such as a pogo pin. Each of the other contacts is a ring contact which contacts the capsule in a circle about the capsule so that a contact is made between a stud and a ring contact regardless of capsule rotational orientation. In the example of Fig. IB, the contact is a sheet of metal cut so that a series of spokes extend radially inward from an annular outer ring and terminate on a circle about the capsule housing at the longitude of one of the feedthrough connectors (studs). Each spoke can independently flex as a cantilever.

The connectors may be rigid instead of flexible if they are mounted on a compliant structure. For example, in Fig. 1C, the ring contacts are stacked on a support with compliant spacers. The spacers provide electrical insulation. They could be made from an elastomer such as silicone. The compliant spacers could be plastic springs or metal springs with an insulating surface to mate with an electrode.

The ring contacts in Fig. 1A lie in a plane, prior to deflection under the force of the capsule, but they could be formed in other shapes with approximately circular openings including cylinders or conic frustums. The contacts may themselves be flexible or they may be rigid but mounted on compliant supports. Some degree of compliance is required so that sufficient contact pressure is made between all of the capsule contacts and the docking station contacts without overly tight dimensional tolerances on the contacts and extreme positional control of the capsule. One of the docking station contacts can be rigid, if desired. The capsule must be pressed down onto the contacts to achieve the desired contact pressure. The docking station may have a capsule cover which presses on the capsule. The force may come from the mass of the cover or may be provided by a spring or clamp. The cover may be hinged, tethered, or fully detachable. The cover may latch onto the docking station housing by bayonet, screw thread, snap, hook, magnet, or other mechanism. If the cover has sufficient mass, no latch mechanism is required.

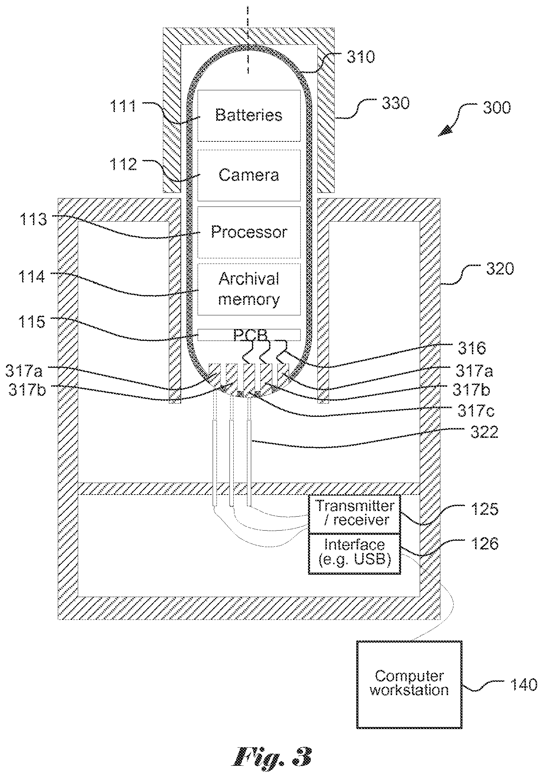

An alternative embodiment is shown in Fig. 3, where the capsule camera 310 is shown in docked position in the docking station with a docking base 320 and a cover 330. The contacts 317a-c on the capsule have approximate circular symmetry, while the contacts in the docking station do not. The system is similar to the system shown in Fig. 1A and the same components are designated by the same reference numbers. The center contact (317c) appears as a circle and the outer ones (317a and 317b) are concentric rings, like an arhery target, when viewed from the end of the capsule. Of course both sets of contacts could have approximate circular symmetry. In the example shown, annular feedthrough contacts 317a-c penetrate the capsule. The possible methods of fabrication are similar to those of the embodiment in Fig. 1A. The methods of connecting these contacts to the electronic system within the capsule are also equivalent, including the possibility that the annular contacts themselves have compliant members that contact electrodes mounted on the PCB or pads on the PCB. For example, flexible interconnect 316 is shown in Fig. 3.

The contacts in the docking station are shown as spring-loaded pins 322 (pogo pins). Other types of compliant contacts could be utilized. One of the docking station contacts could be noncompliant.

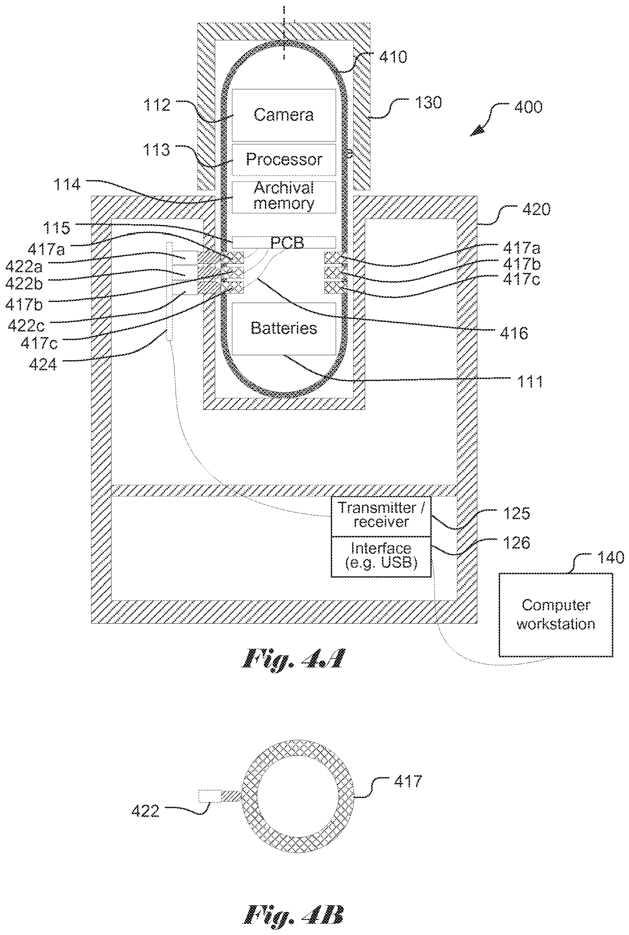

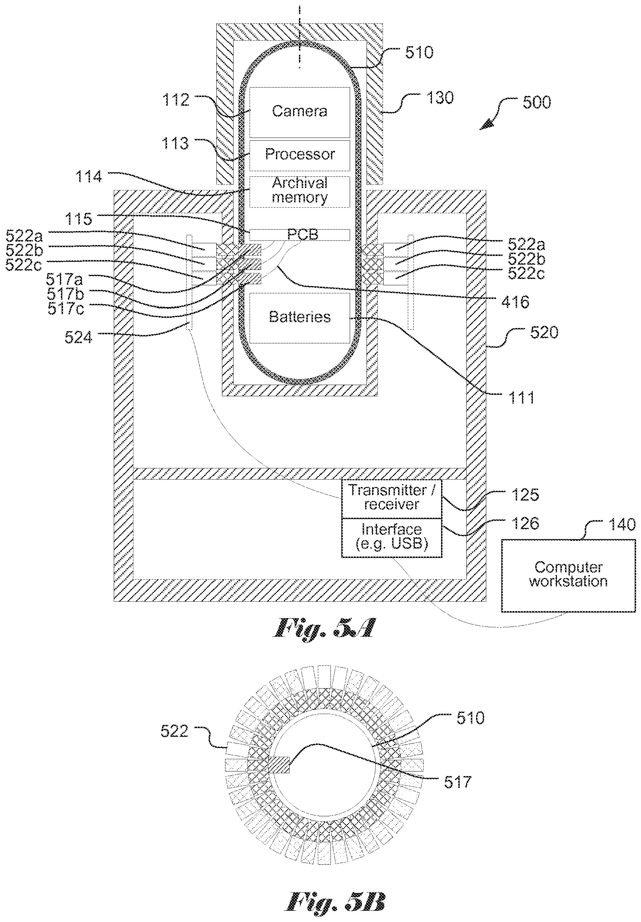

The capsule feedthrough contacts need not be located at the tip of the capsule but could be at other locations. Figs. 4A-4B illustrates an example not part of the present invention, with annular contacts around the middle of the capsule, where the capsule camera 410 is shown in docked position inside the docking station 400 comprising a base part 420 and a cover 430. In the docking station, these could be contacted by plunger contacts or other spring-loaded contacts. Alternatively, stud contacts 422a-c could be positioned at different longitudes on the side of the capsule and the docking station could have mating ring contacts 417a-c. Fig. 4B shows a bottom view of the stud contacts 422a-c and the annular feedthrough 417a-c. In the exemplary docking station, a PCB 424 is used to provide connectivity from the stud contacts 422a-c to the
transmitter/receiver 125. However, other connection means such as direct wiring may be used. The docking station might have multiple plunger contacts 522a-c arrayed in a circle about the capsule at angular intervals Θ and the capsule might have a feedthrough contact 517a-c that covers an angle of at least Θ about the capsules circumference as shown in Figs. 5A-5B. In Fig. 5A, the capsule camera 510 is shown in a docked position inside the docking station 500 comprising a base part 520 and a cover 530. Fig. 5B illustrates a bottom view for one set of plungers 522a-c and one pin feedthrough 517a-c.

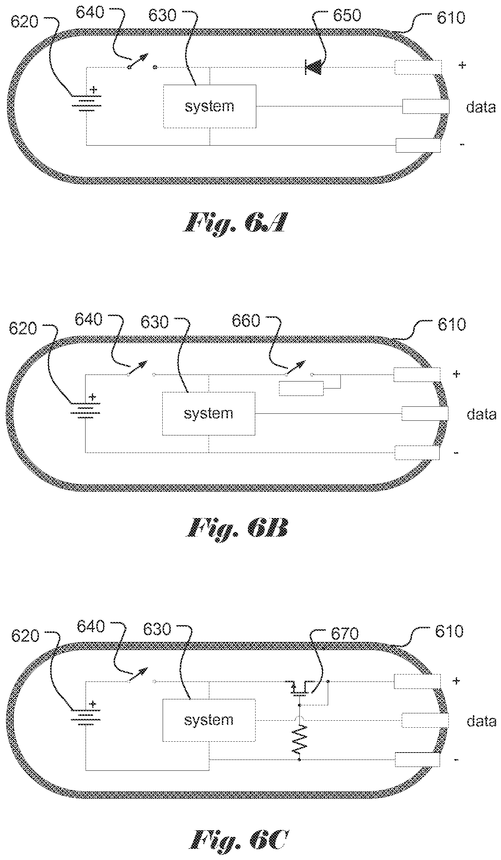

Figs. 6A-6C shows three examples of schematics of the capsule incorporating embodiments of the present invention to control the power in the capsule endoscope. In typical capsule designs the battery is connected to the capsule system by a magnetically actuated switch 640. The capsule package contains a magnet that keeps the switch open until the capsule is removed from the package. The capsule of this invention has feedthrough contacts so that power can be supplied to the capsule when it is outside the body when the battery is likely to have been exhausted. These contacts might also be used to recharge the battery for capsule reuse. In this embodiment, another contact is included for data transfer.

When the capsule is inside the body, the magnetically actuated switch is closed and the battery is connected to the system. However, the battery should not be connected to both of the feedthrough contacts. Otherwise a current could flow through the bodily tissues or through liquids in the GI tract. These currents would drain the battery and could cause local heating, which could be a safety concern. Also, if the capsule is in the stomach, the HCl is corrosive and a voltage applied to the terminal will accelerate any corrosion of the terminal that might occur should the protective noble-metal plating on the contacts be damaged prior to swallowing the capsule.

Fig. 6A illustrates one embodiment of the embodiment, where a diode is connected between one or more of the power contacts and the system electronics. As shown in Fig. 6A, the capsule camera 610a comprises batteries 620 and electronic system 630. The electronic system includes all components that require power source to operate. The diode allows power to be delivered to the capsule from the outside, but not the reverse. The diode might be a PN junction diode, a Schottky diode, or any other type of diode, or any electrical component which provides rectification between two of its terminals. Other electronics could perform a similar function. For example, a switch 660 as shown in Fig. 6B, could be used instead of a diode. The switch could be activated by a voltage applied to the power contacts outside the capsule. The switch would be off without this externally applied voltage and no current would flow from the battery outside the capsule from positive to negative terminal. For example, the switch might comprise an NMOS transistor 670with the gate and drain connected to the positive feedthrough contact as shown in Fig. 6C. A switch could be placed in the path from the negative terminal to the system instead of from the positive terminal to the system. The docking station may use another means to close the switch. It may contain a magnet and the switch may be magnetically actuated such that the magnet closes the switch when the capsule is docked in the docking station. Alternatively, the docking station may provide a signal to the capsule, for example an optical signal, which causes the switch to close.

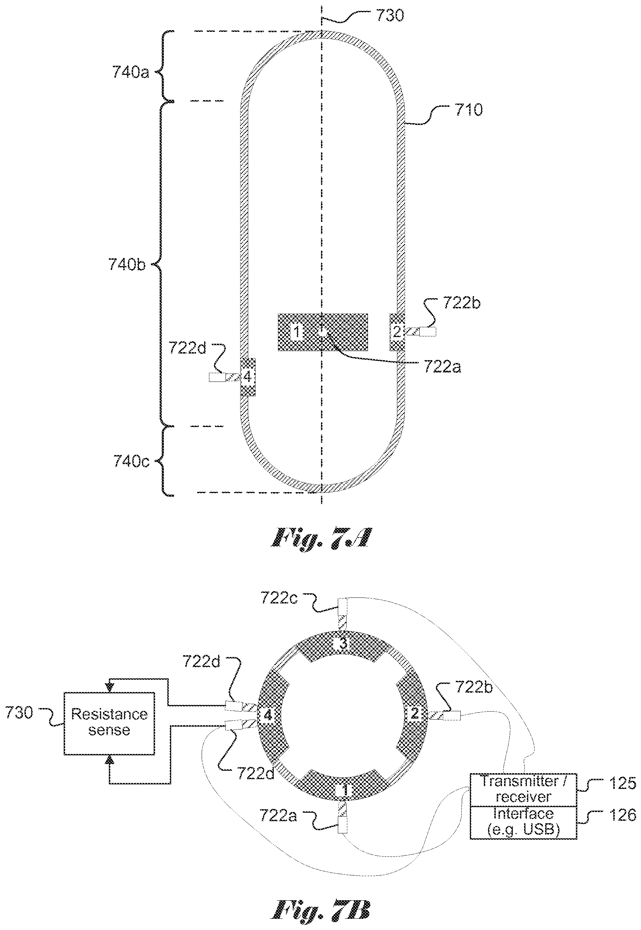

If the capsule and the docking station contacts both lack circular symmetry, the capsule must be rotationally aligned so that the correct connections are made. The capsule endoscope (700) in Fig. 7A illustrates a typical capsule with elongated shape. The longitudinal axis of the capsule endoscope is indicated by dashed line 730. There are two ends (740a and 740c) in the longitudinal direction and a wall section (740b) in the middle. The wall section has a cylinder shape. In Figs. 7A-7B, the capsule 710 has four contacts about its circumference. Three contacts (numbered 1, 2, and 3) lie at the same capsule longitude (overlapping a common lateral plane) and the forth (numbered 4) is at a second longitude (not overlapping that lateral plane). Plunger contacts are arrayed in four locations about the capsule. The docking station contacts 722d to the fourth capsule contact comprise two plungers, while other contacts 722a-c of the docking station may have only one plunger. The correct alignment is made when both plungers contact the fourth capsule contact. When this is the case the electrical resistance 730 between the two plungers is low, and this condition may be sensed. The condition of correct alignment generates a control signal for the alignment process. The control signal could be supplied to a motion controller that rotates the capsule or the contacts relative to the capsule. The control signal might activate an indicator such as a light or a sound that would signal an operator to stop manual rotation of the capsule.

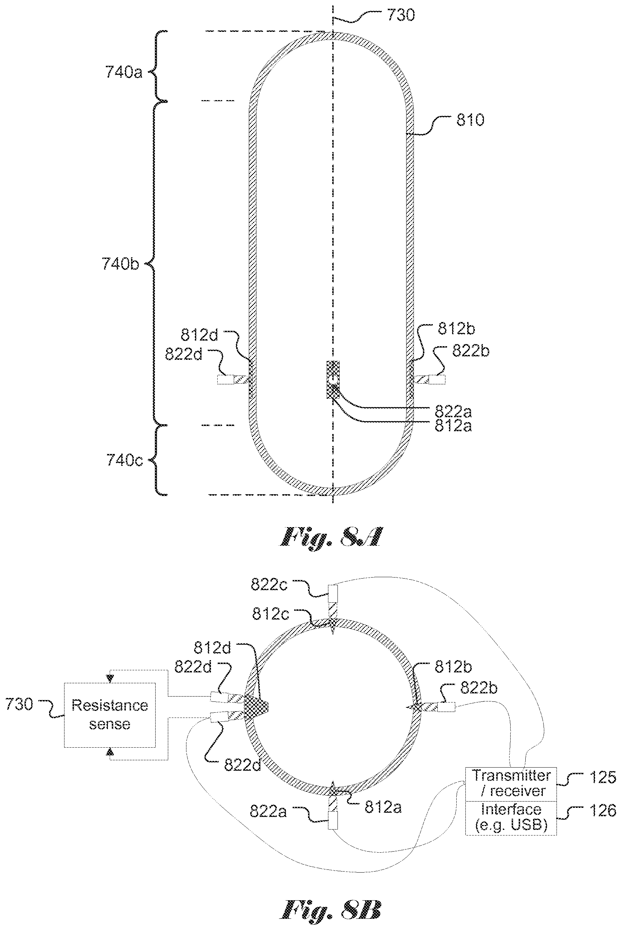

In Figs. 8A-8B, a slightly different embodiment not part of the present invention is shown. All the capsule contacts 812a-d overlap a lateral plane at a given longitude, but the contacts are of different arc lengths. The contact 812d has a longer arc length than the other three. Only this contact is large enough for both of the plungers 822d to touch a contact (i.e., 812d) at the same time. For other contacts, only one plunger (822a-c) is intended to make the connection. The resistance sensed by the resistance sensor 730 is low only for this alignment of the capsule, and this alignment produces the desired connection to the capsule

The above description is presented to enable a person of ordinary skill in the art to practice the present invention as provided in the context of a particular application and its requirements. Various modifications to the described embodiments will be apparent to those with skill in the art, and the general principles defined herein may be applied to other embodiments. Therefore, the present invention is not intended to be limited to the particular embodiments shown and described, but is to be accorded the widest scope consistent with the principles and novel features herein disclosed. In the above detailed description, various specific details are illustrated in order to provide a thorough understanding of the present invention. Nevertheless, it will be understood by those skilled in the art that the present invention may be practiced.

The described examples are to be considered in all respects only as illustrative and not restrictive. The scope of the invention is, therefore, indicated by the appended claims rather than by the foregoing description.

## Claims

1. A capsule endoscopic system comprising:
a capsule endoscope, wherein the capsule endoscope comprises,
a capsule housing (110, 210, 310, 410, 510, 610); and
first circuits (115, 630) inside the capsule housing;
a docking station to interface with the capsule endoscope, wherein the docking station comprises a docking bay (150) to receive the capsule endoscope;
second circuits (125); and
contact means to cause the first circuits to connect to the second circuits when the capsule endoscope is docked in the docking bay in first rotational orientation and second rotational orientation, wherein the contact means comprises,
one or more first contacts (117a/b/c, 217a/b/c, 317a/b/c, 417a/b/c, 517a/b/c) disposed fixedly on or through the capsule housing while maintaining the capsule housing water-tight, wherein said one or more first contacts are connected to the first circuits, and wherein said one or more first contacts comprise one or more feedthrough connectors (117a/b/c, 217a/b/c, 317a/b/c), and said one or more feedthrough connectors (117a/b/c) comprise studs; and
one or more second contacts (122/123, 322, 422a/b/c, 522a/b/c) in the docking stations, wherein said one or more second contacts are coupled to the second circuits; and wherein said one or more second contacts are configured to contact said one or more first contacts and to cause the first circuits to connect to the second circuits when the capsule endoscope is docked in the first rotational orientation and the second rotational orientation, wherein
said one or more second contacts (122/123) comprise one or more annular connectors, configured to be symmetric around a longitudinal axis through a center of the capsule endoscope and **characterised in that**
said one or more annular connectors (122/123) comprise a series of spokes extended radially inward from an annular outer ring.

2. The capsule endoscopic system of Claim 1, wherein said one or more feedthrough connectors (117a/b/c, 217a/b/c, 317a/b/c) are located at one end of the capsule endoscope.

3. The capsule endoscopic system of Claim 2, wherein said one or more feedthrough connectors (117a/b/c, 317a/b/c) are substantially flush with exterior surface of the capsule housing.

4. The capsule endoscopic system of Claim 2, wherein said one or more feedthrough connectors (317a/b/c) or said one or more second contacts (122/123) are configured to be circular symmetric around a longitudinal axis through a center of the capsule endoscope.

5. The capsule endoscopic system of Claim 4, wherein said one or more feedthrough connectors (317a/b/c) comprise one or more concentric ring connectors.

6. The capsule endoscopic system of Claim 5, wherein said one or more feedthrough connectors (117a/b/c) comprise a center connector located at a center of said one or more concentric ring connectors (122/123).

7. The capsule endoscopic system of Claim 1, wherein said one or more first contacts (417a/b/c, 517a/b/c, 812a/b/cd) comprise one or more feedthrough connectors located at a wall section of the capsule endoscope.

8. The capsule endoscopic system of Claim 7, wherein said one or more feedthrough connectors (417a/b/c) or said one or more second contacts (522a/b/c) are configured to be circular symmetric around a longitudinal axis through a center of the capsule endoscope.

9. The capsule endoscopic system of Claim 8, wherein said one or more feedthrough connectors comprise studs (517a/b/c).

10. The capsule endoscopic system of Claim 8, wherein said one or more feedthrough connectors (417a/b/c) comprise one or more concentric ring connectors.

11. The capsule endoscopic system of Claim 1, further comprising a battery (620), and a diode (650), a switch (660) or an NMOS transistor (670) inside the capsule housing, wherein power is provided from or through the docking station to the capsule endoscope through a first connector and a second connector, and the diode or the NMOS transistor is connected to the first connector so as to block current from flowing from the battery to the first connector while allowing current to flow from the first connector to the first circuits.

12. The capsule endoscopic system of Claim 1, further comprising a battery (620) and a switch (640) inside the capsule housing, wherein power is provided from the docking station to the capsule endoscope through a first connector and a second connector, and the switch is connected to the first connector so as to block current from flowing from the battery to the first connector while the capsule endoscope is not docked in the docking station.

13. The capsule endoscopic system of Claim 12, wherein the switch (640) is closed by applying a voltage between the first connector and the second connector.

14. The capsule endoscopic system of Claim 12, wherein a magnet in the docking station causes the switch to close.

15. The capsule endoscopic system of Claim 12, wherein the docking station provides a signal to the capsule endoscope which causes the switch to close.

16. The capsule endoscopic system of Claim 1, wherein the first rotational orientation and the second rotational orientation correspond to any rotational orientation within 360 degrees.

17. The capsule endoscopic system of Claim 1, wherein the first circuits comprise non-volatile memory to store images, data or both, wherein the images, data or both are accessed by the second circuits when the capsule endoscope is docked in the docking station.

18. The capsule endoscopic system of Claim 1, wherein the second circuits (125) comprise a signal transmitter, a signal receiver, or a combination thereof.

19. The capsule endoscopic system of Claim 1, wherein the second circuits are inside the docking station or external to the docking station.

## Patentansprüche

1. Ein Kapselendoskop-System, umfassend:
ein Kapselendoskop, wobei das Kapselendoskop umfasst,
ein Kapselgehäuse (110, 210, 310, 410, 510, 610); und
erste Schaltungen (115, 630) im Inneren des Kapselgehäuses;
eine Dockingstation zur Verbindung mit dem Kapselendoskop, wobei die Dockingstation eine Andockstation (150) zur Aufnahme des Kapselendoskops umfasst;
zweite Schaltungen (125); und
Kontaktmittel, um zu bewirken, dass die ersten Schaltungen mit den zweiten Schaltungen verbunden werden, wenn das Kapselendoskop in der Andockstation in einer ersten Drehrichtung und einer zweiten Drehrichtung angedockt ist, wobei das Kontaktmittel umfasst,
einen oder mehrere erste Kontakte (117a/b/c, 217a/b/c, 317a/b/c, 417a/b/c, 517a/b/c), die fest am oder durch das Kapselgehäuse angeordnet sind, während das Kapselgehäuse wasserdicht gehalten wird, wobei der eine oder die mehreren ersten Kontakte mit den ersten Schaltungen verbunden sind, und wobei der eine oder die mehreren ersten Kontakte einen oder mehrere Durchführungsverbinder (117a/b/c, 217a/b/c, 317a/b/c) umfassen, und der eine oder die mehreren Durchführungsverbinder (117a/b/c) Bolzen umfassen; und
einen oder mehrere zweite Kontakte (122/123, 322, 422a/b/c, 522a/b/c) in den Dockingstationen, wobei der eine oder die mehreren zweiten Kontakte mit den zweiten Schaltungen verbunden sind, und wobei der eine oder die mehreren zweiten Kontakte so konfiguriert sind, um den einen oder die mehreren ersten Kontakte zu kontaktieren und zu bewirken, dass die ersten Schaltungen mit den zweiten Schaltungen verbunden werden, wenn das Kapselendoskop in der Andockstation in der ersten Drehrichtung und der zweiten Drehrichtung angedockt ist, wobei der eine oder die mehreren zweiten Kontakte (122/123) einen oder mehrere Ringverbinder umfassen, die so die so konfiguriert sind, dass sie um eine Längsachse durch eine Mitte des Kapselendoskops symmetrisch sind, und **dadurch gekennzeichnet, dass** der eine oder die mehreren Ringverbinder (122/123) eine Reihe von Speichen umfassen, die sich radial nach innen von einem ringförmigen Außenring erstrecken.

2. Das Kapselendoskop-System nach Anspruch 1, wobei sich der eine oder die mehreren Durchführungsverbinder (117a/b/c, 217a/b/c, 317a/b/c) an einem Ende des Kapselendoskops befinden.

3. Das Kapselendoskop-System nach Anspruch 2, wobei der eine oder die mehreren Durchführungsverbinder (117a/b/c, 317a/b/c) im Wesentlichen bündig mit der Außenfläche des Kapselgehäuses sind.

4. Das Kapselendoskop-System nach Anspruch 2, wobei der eine oder die mehreren Durchführungsverbinder (317a/b/c) oder der eine oder die mehreren zweiten Kontakte (122/123) konfiguriert sind, um kreissymmetrisch um eine Längsachse durch eine Mitte des Kapselendoskops zu sein.

5. Das Kapselendoskop-System nach Anspruch 4, wobei der eine oder die mehreren Durchführungsverbinder (317a/b/c) einen oder mehrere konzentrische Verbindungsringe umfassen.

6. Das Kapselendoskop-System nach Anspruch 5, wobei der eine oder die mehreren Durchführungsverbinder (117a/b/c) einen zentralen Verbinder umfassen, der sich in der Mitte des einen oder der mehreren konzentrischen Ringverbinder (122/123) befindet.

7. Das Kapselendoskop-System nach Anspruch 1, wobei der eine oder die mehreren ersten Kontakte (417a/b/c, 517a/b/c, 812a/b/cd) einen oder mehrere Durchführungsverbinder umfassen, die sich an einem Wandabschnitt des Kapselendoskops befinden.

8. Das Kapselendoskop-System nach Anspruch 7, wobei der eine oder die mehreren Durchführungsverbinder (417a/b/c) oder der eine oder die mehreren zweiten Kontakte (522a/b/c) konfiguriert sind, um kreissymmetrisch um eine Längsachse durch eine Mitte des Kapselendoskops zu sein.

9. Das Kapselendoskop-System nach Anspruch 8, wobei der eine oder die mehreren Durchführungsverbinder Bolzen (517a/b/c) umfassen.

10. Das Kapselendoskop-System nach Anspruch 8, wobei der eine oder die mehreren Durchführungsverbinder (417a/b/c) einen oder mehrere konzentrische Ringverbinder umfassen.

11. Das Kapselendoskop-System nach Anspruch 1, ferner umfassend eine Batterie (620) und eine Diode (650), einen Schalter (660) oder einen NMOS-Transistor (670) im Inneren des Kapselgehäuses, wobei Strom von oder durch die Dockingstation zum Kapselendoskop über einen ersten Verbinder und einen zweiten Verbinder bereitgestellt wird, und die Diode oder der NMOS-Transistor mit dem ersten Verbinder verbunden ist, um den Stromfluss von der Batterie zum ersten Verbinder zu blockieren, während der Strom vom ersten Verbinder zu den ersten Schaltungen fließen kann.

12. Das Kapselendoskop-System nach Anspruch 1, ferner umfassend eine Batterie (620) und einen Schalter (640) im Inneren des Kapselgehäuses, wobei Strom von oder durch die Dockingstation zum Kapselendoskop über einen ersten Verbinder und einen zweiten Verbinder bereitgestellt wird, und der Schalter mit dem ersten Verbinder verbunden ist, um den Stromfluss von der Batterie zum ersten Verbinder zu blockieren, während das Kapselendoskop nicht in der Dockingstation dockt.

13. Das Kapselendoskop-System nach Anspruch 12, wobei der Schalter (640) durch Anlegen einer Spannung zwischen dem ersten Verbinder und dem zweiten Verbinder geschlossen wird.

14. Das Kapselendoskop-System nach Anspruch 12, wobei ein Magnet in der Dockingstation das Schließen des Schalters bewirkt.

15. Das Kapselendoskop-System nach Anspruch 12, wobei die Dockingstation ein Signal an das Kapselendoskop liefert, das das Schließen des Schalters bewirkt.

16. Das Kapselendoskop-System nach Anspruch 1, wobei die erste Drehrichtung und die zweite Drehrichtung jeder Drehrichtung innerhalb von 360 Grad entsprechen.

17. Das Kapselendoskop-System nach Anspruch 1, wobei die ersten Schaltungen einen nichtflüchtigen Speicher zum Speichern von Bildern, Daten oder beidem umfassen, wobei die Bilder, Daten oder beides von den zweiten Schaltungen abgerufen werden, wenn das Kapselendoskop in der Dockingstation angedockt ist.

18. Das Kapselendoskop-System nach Anspruch 1, wobei die zweiten Schaltungen (125) einen Signalsender, einen Signalempfänger oder eine Kombination davon umfassen.

19. Das Kapselendoskop-System nach Anspruch 1, wobei sich die zweiten Schaltungen innerhalb der Dockingstation oder außerhalb der Dockingstation befinden.

## Revendications

1. Système endoscopique à capsule comprenant :
un endoscope à capsule, dans lequel l'endoscope à capsule comprend,
un logement de capsule (110, 210, 310, 410, 510, 610) ; et
des premiers circuits (115, 630) à l'intérieur du logement de capsule ;
une station d'ancrage pour faire l'interface avec l'endoscope à capsule, dans lequel la station d'ancrage comprend une baie d'ancrage (150) pour recevoir l'endoscope à capsule ;
des seconds circuits (125) ; et
des moyens de contact pour amener les premiers circuits à se connecter aux seconds circuits quand l'endoscope à capsule est ancré dans la baie d'ancrage dans une première orientation rotative et une seconde orientation rotative, dans lequel les moyens de contact comprennent,
un ou plusieurs premiers contacts (117a/b/c, 217a/b/c, 317a/b/c, 417a/b/c, 517a/b/c) disposés à demeure sur ou à travers le logement de capsule tout en maintenant le logement de capsule étanche, dans lequel lesdits un ou plusieurs premiers contacts sont connectés aux premiers circuits, et dans lequel lesdits un ou plusieurs premiers contacts comprennent un ou plusieurs connecteurs de traversée (117a/b/c, 217a/b/c, 317a/b/c), et lesdits un ou plusieurs connecteurs de traversée (117a/b/c) comprennent des montants ; et
un ou plusieurs seconds contacts (122/123, 322, 422a/b/c, 522a/b/c) dans les stations d'ancrage, dans lequel lesdits un ou plusieurs seconds contacts sont couplés aux seconds circuits ; et dans lequel lesdits un ou plusieurs seconds contacts sont configurés pour entrer en contact avec lesdits un ou plusieurs premiers contacts et pour amener les premiers circuits à se connecter aux seconds circuits quand l'endoscope à capsule est ancré dans la première orientation rotative et la seconde orientation rotative, dans lequel lesdits un ou plusieurs seconds contacts (122/123) comprennent un ou plusieurs connecteurs annulaires, configurés pour être symétriques autour d'un axe longitudinal à travers un centre de l'endoscope à capsule et **caractérisé en ce que** lesdits un ou plusieurs connecteurs annulaires (122/123) comprennent une série de rayons étendus radialement vers l'intérieur depuis une bague extérieure annulaire.

2. Système endoscopique à capsule selon la revendication 1, dans lequel lesdits un ou plusieurs connecteurs de traversée (117a/b/c, 217a/b/c, 317a/b/c) se situent au niveau d'une extrémité de l'endoscope à capsule.

3. Système endoscopique à capsule selon la revendication 2, dans lequel lesdits un ou plusieurs connecteurs de traversée (117a/b/c, 317a/b/c) sont sensiblement à fleur avec une surface extérieure du logement de capsule.

4. Système endoscopique à capsule selon la revendication 2, dans lequel lesdits un ou plusieurs connecteurs de traversée (317a/b/c) ou lesdits un ou plusieurs seconds contacts (122/123) sont configurés pour être circulairement symétriques autour d'un axe longitudinal à travers un centre de l'endoscope à capsule.

5. Système endoscopique à capsule selon la revendication 4, dans lequel lesdits un ou plusieurs connecteurs de traversée (317a/b/c) comprennent un ou plusieurs connecteurs de bague concentriques.

6. Système endoscopique à capsule selon la revendication 5, dans lequel lesdits un ou plusieurs connecteurs de traversée (117a/b/c) comprennent un connecteur central situé au niveau d'un centre desdits un ou plusieurs connecteurs de bague concentriques (122/123).

7. Système endoscopique à capsule selon la revendication 1, dans lequel lesdits un ou plusieurs premiers contacts (417a/b/c, 517a/b/c, 812a/b/c) comprennent un ou plusieurs connecteurs de traversée situés au niveau d'une section de paroi de l'endoscope à capsule.

8. Système endoscopique à capsule selon la revendication 7, dans lequel lesdits un ou plusieurs connecteurs de traversée (417a/b/c) ou lesdits un ou plusieurs seconds contacts (522a/b/c) sont configurés pour être circulairement symétriques autour d'un axe longitudinal à travers un centre de l'endoscope à capsule.

9. Système endoscopique à capsule selon la revendication 8, dans lequel lesdits un ou plusieurs connecteurs de traversée comprennent des goujons (517a/b/c).

10. Système endoscopique à capsule selon la revendication 8, dans lequel lesdits un ou plusieurs connecteurs de traversée (417a/b/c) comprennent un ou plusieurs connecteurs de bague concentriques.

11. Système endoscopique à capsule selon la revendication 1, comprenant en outre une batterie (620), et une diode (650), un commutateur (660) ou un transistor NMOS (670) à l'intérieur du logement de capsule, dans lequel de l'énergie est fournie depuis ou à travers la station d'ancrage à l'endoscope à capsule à travers un premier connecteur et un second connecteur, et la diode ou le transistor NMOS est connecté au premier connecteur de manière à bloquer l'écoulement de courant de la batterie vers le premier connecteur tout en permettant au courant de s'écouler depuis le premier connecteur vers les premiers circuits.

12. Système endoscopique à capsule selon la revendication 1, comprenant en outre une batterie (620) et un commutateur (640) à l'intérieur du logement de capsule, dans lequel de l'énergie est fournie depuis la station d'ancrage à l'endoscope à capsule à travers un premier connecteur et un second connecteur, et le commutateur est connecté au premier connecteur de manière à bloquer l'écoulement de courant de la batterie vers le premier connecteur alors que l'endoscope à capsule n'est pas ancré dans la station d'ancrage.

13. Système endoscopique à capsule selon la revendication 12, dans lequel le commutateur (640) est fermé en appliquant une tension entre le premier connecteur et le second connecteur.

14. Système endoscopique à capsule selon la revendication 12, dans lequel un aimant dans la station d'ancrage entraîne la fermeture du commutateur.

15. Système endoscopique à capsule selon la revendication 12, dans lequel la station d'ancrage fournit un signal à l'endoscope à capsule qui entraîne la fermeture du commutateur.

16. Système endoscopique à capsule selon la revendication 1, dans lequel la première orientation rotative et la seconde orientation rotative correspondent à toute orientation rotative dans les 360 degrés.

17. Système endoscopique à capsule selon la revendication 1, dans lequel les premiers circuits comprennent une mémoire non volatile pour stocker des images, des données, ou les deux, dans lequel les seconds circuits accèdent aux images, aux données, ou aux deux, quand l'endoscope à capsule est ancré dans la station d'ancrage.

18. Système endoscopique à capsule selon la revendication 1, dans lequel les seconds circuits (125) comprennent un émetteur de signal, un récepteur de signal, ou une combinaison de ceux-ci.

19. Système endoscopique à capsule selon la revendication 1, dans lequel les seconds circuits se trouvent à l'intérieur de la station d'ancrage ou à l'extérieur de la station d'ancrage.
